# EUROPÄISCHE PATENTSCHRIFT

(11) **EP 0 508 194 B1**
(45) Veröffentlichungstag und Bekanntmachung des Hinweises auf die Patenterteilung: **10.12.1997**
(21) Anmeldenummer: 92104944.1
(22) Anmeldetag: 21.03.1992
(51) Int. Cl.: A61K 38/00, A61K 47/00

(54) **Stabilisierte Faktor VIII-Präparationen**
Stabilized factor VIII preparations
Préparations de facteur VIII stabilisées

(30) Priorität: 09.04.1991 DE 4111393
(43) Veröffentlichungstag der Anmeldung: 14.10.1992
(73) Patentinhaber: BEHRINGWERKE Aktiengesellschaft, 35001 Marburg (DE)
(72) Erfinder: Freudenberg, Wilfried, W-3553 Cölbe-Schönstadt (DE)

(56) Entgegenhaltungen:
- EP-A- 0 315 968
- EP-A- 0 412 466
- EP-A- 0 468 181
- WO-A-91/10439
- GB-A- 941 019

## Beschreibung

Die Erfindung betrifft stabilisierte Lösungen mit F VIII-Koagulationsaktivität größer als 1000 IU/mg, Verfahren zu ihrer Herstellung und ihre Verwendung.

Der Gerinnungsfaktor VIII:C (F VIII:C) ist ein Plasmaprotein und wesentlich für den Ablauf des intrinsischen Wegs der Blutgerinnung. Ein Mangel an oder Defekt des Blutgerinnungsfaktors VIII:C führt zu einer lebensgefährdenden Störung der Blutgerinnung, der Haemophilie A. Zur Therapie der Haemophilie A werden Konzentrate des F VIII:C aus Humanplasma oder gentechnisch hergestellter F VIII:C eingesetzt.

Diese F VIII-Präparate unterscheiden sich bezüglich ihrer Reinheit, d.h. dem Vorhandensein von nicht gerinnungsaktiven Proteinen neben dem Wirkstoff F VIII:C. Als ein "Very High Purity"-F VIII (VHP-F VIII:C) wird ein F VIII bezeichnet, der mehr als 1000 U/mg vor einer Stabilisierung mit Albumin aufweist (WHO, Expert Committee on Biological Standardization).

Solche VHP-F VIII:C haben potentielle Vorteile bei der Behandlung der Haemophilie. Diese sind die Virusfreiheit und ein sehr geringer Gehalt an Fremdprotein, wodurch nach Gabe dieser Konzentrate das Immunsystem der Patienten weniger stark belastet wurde. Der an sich mögliche Vorteil, durch Gabe einer F VIII-Präparation mit hoher spezifischer Aktivität das Immunsystem eines haemophilen Patienten weniger stark zu belasten, wird jedoch dadurch zunichte gemacht, daß man zur Stabilisierung des VHP-F VIII hohe Albumin-Konzentrationen dem hochgereinigten

Präparat zusetzt. Durch diesen Zusatz an Albumin erreichen die hochgereinigten F VIII-Konzentrate in ihrer Endformulierung spezifische Aktivitäten von nur 3 - 10 U/mg.

Wenngleich der Zusatz von Albumin in Bezug auf die Virussicherheit nur eine geringe Gefahr birgt, so ist jedoch zu bedenken, daß bei der durchschnittlichen Reinheit des Albumin von 95 % wiederum unerwünschte Begleitproteine dem Patienten appliziert werden, die sein Immunsystem belasten können.

Es sind "High Purity" F VIII-Präparate bekannt, die auf einen Zusatz von Albumin zur Stabilisierung des F VIII verzichten (Schwinn, Smith & Wolter, Drug Res. 39 (1989), 1302). Diese Präparate erreichen spezifische Aktivitäten von ca. 100 U/mg Protein. Bezogen auf eine maximal erreichbare F VIII-Aktivität von ca. 5000 U/mg bedeutet das, daß nur ca. 2 % des Proteingehaltes dieser Präparationen aus F VIII:C Protein bestehen. Eine Stabilisierung dieser 2 % F VIII:C durch die 98 % Begleitproteine ist hier anzunehmen, da ein großer Teil dieser Begleitproteine dem von Willebrand-Faktor (vWF) zuzurechnen sind. Bekanntermaßen hat der von Willebrand-Faktor eine stabilisierende Wirkung auf den F VIII:C.

Anders bei Very High Purity Präparaten mit spezifischen Aktivitäten, die vor Albuminstabilisierung gewöhnlich mehr als 25fach höher als bei High Purity Präparaten liegen, und deren Gehalt an vWF sehr gering ist. Dieser geringe Anteil an vWF kann nicht mehr für eine ausreichende F VIII-Stabilisierung sorgen, so daß die F VIII-Aktivität in nicht mit Albumin stabilisierten Lösungen rasch abnimmt.

Die Aufgabe der vorliegenden Erfindung war es daher, ein Verfahren zur Verfügung zu stellen, das die Herstellung einer hochkonzentrierten, physiologisch verträglichen Lösung eines VHP-F VIII:C-Präparates erlaubt, welche keinen Zusatz von Proteinen zur Stabilisierung benötigt.

In der EP-A-0 315 968 sind FVIII-C-Lösungen mit einer spezifischen Aktivität von mehr als 1000 IU/mg mit stabilisierenden Zusätzen eines Kohlenhydrates, Lysin und Histidin in Konzentrationen von 0.0001 bis 10 mol/l beschrieben. Stabilisierung von FVIII:C-Lösungen einer spezifischen Aktivität von 56 bis 215 IE/mg durch Kohlenhydrat und Glycin in einer Konzentration von 3 % (d.h. 0.4 mol/l) ist aus der EP-A-0 412 466 entnehmbar. In WO-A-9110439 schließlich wird eine injizierbare FVIII:C-Lösung beschrieben, die eine oder mehrere Aminosäuren in einer Konzentration von 0.1 bis 1.0 mol/l sowie ein Kohlenhydrat enthält.

Es wurde gefunden, daß Arginin oder eines seiner Salze, besonders in Kombination mit Glycin, mit gegebenenfalls einem Detergenz und/oder organischem Polymer besonders gut FVIII:C-Lösungen mit spezifischer Aktivität größer 1000 IU/mg zu stabilisieren vermögen.

Eine gute Stabilisierung wird erreicht durch Kombination von Arginin oder eines Salzes oder oder mit einem nicht-ionischen Detergenz wie ^{R}Polysorbate 20 oder ^{R}Polysorbate 80 oder einem organischen Polymer wie Polyethylenglykol 1500.

Als besonders geeignet zur Herstellung einer stabilen, albumin-freien VHP-F VIII:C-Lösung hat sich eine Kombination der Aminosäuren Arginin und Glycin, bevorzugt 0.01 bis 1 mol/l mit dem nicht-ionischen Detergenz ^{R}Tween 80, bevorzugt 0.001 bis 0.5 % (v/v) und einem Neutralzucker wie Saccharose, bevorzugt 0.1 bis 10 % gezeigt.

Der pH-Wert einer solchen Lösung wird mittels einer organischen Säure, bevorzugt 10 %ige Essigsäure, zwischen pH 5.5 und 8.5, bevorzugt zwischen pH 6.5 und 7.5 eingestellt.

Gegenstand der Erfindung ist auch ein Arzneimittel enthaltend eine solche Lösung. Dieses Arzneimittel kann außer einer solchen Lösung übliche, pharmazeutisch verträgliche, stabilisierende und/oder puffernde Substanzen enthalten, besonders ein Kohlenhydrat.

Gegenstand der Erfindung ist ebenfalls ein Verfahren zur Herstellung einer solchen Lösung, wobei einer Lösung mit Faktor VIII:C-Aktivität Arginin oder eines seiner Salze oder Derivate und gegebenenfalls ein organisches Polymer oder ein Detergenz zugesetzt wird.

Die vorteilhafte Wirkung des erfindungsgemäßen Verfahrens kann beispielsweise für eine über Chromatographie an monoklonalen Anti-F VIII:C-Antikörpern gereinigte F VIII:C-Präparation gezeigt werden, wobei der F VIII:C sowohl aus Plasma gewonnen als auch gentechnisch z.B. in CHO (Chinese Hamster Ovary)-Zellen hergestellt sein kann. Dabei wird das Eluat der monoklonalen Antikörpersäule beispielsweise zu gleichen Teilen mit einer Lösung der obengenannten Substanzen versetzt und anschließend gegen diese Lösung dialysiert. Die so erhaltene stabilisierte F VIII:C-Präparation läßt sich unter geringen methoden-bedingten Verlusten sterilfiltrieren und abfüllen. Ein Lyophilisat dieser so erhaltenen Präparation zeigt nach Lösen unverändert hohe F VIII:C-Aktivitäten.

Mit dem erfindungsgemäßen Verfahren kann eine VHP-F VIII:C-Präparation hergestellt werden, deren spezifische Volumenaktivität mindestens 200 IU/ml beträgt, bei einer spezifischen Aktivität von größer als 2000 IU/mg. Diese Konzentration gewährleistet eine problemlose Handhabung durch geringe zu applizierende Volumen.

Eine solche Präparation bedarf keiner weiteren Stabilisierung durch Proteine, wodurch die Gefahr von Viruskontaminationen vermieden wird. Zugleich wird durch die Verringerung der hohen Proteinfracht, die durch die Zugabe des für die Arzneiwirkung unnötigen Albumins und der darin enthaltenden unerwünschten Verunreinigungen das Immunsystem des Patienten wesentlich entlastet.

Da physiologisch verträgliche Substanzen zur Stabilisierung zugesetzt werden, treten bei der Applikation der erfindungsgemäßen Lösung keine Unverträglichkeitsreaktionen auf.

### Beispiel 1:

Es wurden zwei VHP-F VIII:C-Präparationen hergestellt, sowohl mittels Affinitätschromatographie an monoklonalen Anti-vWF-Ig (nach Fulcher & Zimmermann PNAS (1982), 79, 1649) und Dissoziation des vWF/F VIII:C-Komplex durch Lösung mit einer CaCl₂-Konzentration von 300 mM in 0.1 M Acetat, 0.1 M Lysin, pH 6.8 (Eluat I) als auch mittels Chromatographie an monoklonalen Anti-F VIII:C-Ig und Elution des F VIII:C durch 50 % Ethylenglykol in 0.1 M Acetat, 0.1 M Lysin, pH 6.8 (Eluat II). Im Eluat I wurde eine spezifische F VIII:C-Aktivität von 2500 IU/mg und 419 IU/ml und im Eluat II von 3280 IU/mg und 454 IU/ml bestimmt. Beide Eluate wurden jeweils geteilt. Ein Teil wurde je im Volumenverhältnis 1:1 mit einer 1 %igen Human-Albumin-Lösung in 0.75 % Saccharose, 3 % Glycin und 0.1 Mol/l NaCl versetzt (Eluat I_{HSA}, Eluat II_{HSA}). Die jeweils andere Hälfte wurde ebenfalls 1:1 mit dem Stabilisierungspuffer (0.75 % Saccharose, 3 % Glycin, 3 % Arginin, 0.05 % ^{R}Tween 80, pH 6.8) versetzt (Eluat I_{S}, Eluat II_{S}). Die Albumin enthaltenden Proben wurden gegen 0.75 % Saccharose, 3 % Glycin, 0.1 Mol/l NaCl, pH 6.8 dialysiert, die anderen gegen Stabilisierungspuffer. Die Dialyse fand bei 4°C für 16 Stunden und 1000-fachem Volumenwechsel statt. Die F VIII:C-Aktivitäten wurden vor und nach der Dialyse gemessen. Aufgeführt ist in Tabelle 1 die F VIII:C-Aktivität in % bezogen auf die Gesamt-F VIII:C-Aktivität der jeweiligen Probe vor Dialyse.

**Tabelle 1**

| Eluat I_{HSA} | Eluat I_{S} | Eluat II_{HSA} | Eluat II_{S} |
|---|---|---|---|
| 92 | 94 | 94 | 93 |

Die Ergebnisse zeigen, daß eine Stabilisierung der VHP-F VIII:C-Eluate mittels der erfindungsgemäßen Stabilisierungslösung unabhängig von der Präparationsmethode und im gleichen Maße wie durch Albumin-Zusatz erreicht wird.

### Beispiel 2:

Es wurde ein F VIII:C-Eluat nach Immunaffinitätschromatographie an monoklonalen Anti-F VIII:C-Antikörpern mit einer spezifischen F VIII:C-Aktivität von 3860 IU/mg Protein und 462 IU/ml gewonnen. Diese wurde im Volumenverhältnis 1 : 1 mit verschiedenen Stabilisierungslösungen versetzt und gegen die jeweilige Stabilisierungslösung, wie im Beispiel 1 beschrieben, dialysiert. In allen Lösungen wurde ggfs. mit 10 % Essigsäure ein pH-Wert von 6.8 eingestellt.

Folgende Stabilisierungslösungen wurden eingesetzt:
I. 0.75 % Saccharose, 0.4 M Glycin, 0.15 M Natriumchlorid
II. 0.01 M Natriumcitrat, 0.08 M Glycin, 0.016 M Lysin, 0.0025 M Calciumchlorid, 0.4 M Natriumchlorid
III. 1 % Saccharose, 0.14 M Arginin, 0.1 M Natriumchlorid
IV. 1 % Saccharose, 0.4 M Glycin, 0.14 M Arginin, 0.1 M Natriumchlorid, 0.05 % Tween 80

Die F VIII:C-Aktivität wurde vor und nach der Dialyse bestimmt. Aufgetragen in % wurde in Tabelle 2 die F VIII:C-Aktivität nach Dialyse bezogen auf die jeweilige Aktivität vor Dialyse.

**Tabelle 2**

| Ansatz | I | II | III | IV |
|---|---|---|---|---|
| F VIII:C-Aktivität nach 16 Stunden Dialyse | 39,3 % | 35.1 % | 82.4 % | 96.2 % |

Die unter I und II eingesetzten Lösungen können zur Stabilisierung von Albumin-freien HP-F VIII-Präparaten mit spezifischen F VIII:C-Aktivitäten von 100 - 200 IU/mg unter Verzicht auf Albumin-Zusatz eingesetzt werden. Zur Stabilisierung von VHP-F VIII-Präparationen mit spezifischen F VIII:C-Aktivitäten größer als 1000 IU/mg sind die Lösungen III und IV geeignet.

## Patentansprüche (Patentansprüche für folgende(n) Vertragsstaat(en): AT, BE, CH, DE, DK, FR, GB, IT, LI, LU, NL, PT, SE)

1. Eine Lösung mit Faktor VIII:C-Aktivität größer als 1000 IU/mg, die durch Zusatz von Arginin oder seiner Salze stabilisiert wird und gegebenenfalls als weitere Bestandteile ein Detergenz und/oder ein organisches Polymer enthält.

2. Eine Lösung nach Anspruch 1, dadurch gekennzeichnet, daß sie zusätzlich Glycin enthält.

3. Eine Lösung nach Anspruch 1 oder 2, dadurch gekennzeichnet, daß Arginin und Glycin jeweils im Bereich von 0.01 bis 1 mol/l zusammen mit Tween® 80 im Bereich von 0.001 % bis 0.5 % (v/v) und Saccharose im Bereich von 0.1 % bis 10 % enthalten sind.

4. Eine Lösung nach Anspruch 3, dadurch gekennzeichnet, daß 0.14 M Arginin, 0.4 M Glycin, 0.05 % Tween® 80, 1 % Saccharose und 0.1 M Natriumchlorid enthalten sind.

5. Verfahren zur Stabilisierung einer Faktor VIII:CLösung mit Faktor VIII:C-Aktivität größer als 1000 IU/mg, dadurch gekennzeichnet, daß Arginin oder seiner Salze und gegebenenfalls als weitere Bestandteile ein Detergenz und/oder ein organisches Polymer zugesetzt wird.

6. Arzneimittel, enthaltend eine Lösung gemäß den Ansprüchen 1, 2, 3 oder 4.

## Patentansprüche (Patentansprüche für folgende(n) Vertragsstaat(en): ES, GR)

1. Verfahren zur Stabilisierung einer Faktor VIII:CLösung mit Faktor VIII:C-Aktivität größer als 1000 IU/mg, dadurch gekennzeichnet, daß Arginin oder seiner Salze und gegebenenfalls als weitere Bestandteile ein Detergenz und/oder ein organisches Polymer zugesetzt wird.

2. Verfahren nach Anspruch 1, dadurch gekennzeichnet, daß zusätzlich Glycin zugesetzt wird.

3. Verfahren nach Anspruch 1 oder 2 , dadurch gekennzeichnet, daß Arginin und Glycin jeweils im Bereich von 0.01 bis 1 mol/l zusammen mit Tween® 80 im Bereich von 0.001 % bis 0.5 % (v/v) und Saccacharose im Bereich von 0.1 % bis 10 % zugesetzt wird.

4. Verfahren nach Anspruch 3, dadurch gekennzeichnet, daß 0.14 M Arginin, 0.4 M Glycin, 0.05 % Tween® 80 und 1 % Saccharose und 0.1 M Natriumchlorid zugesetzt wird.

## Claims (Claims for the following Contracting State(s): AT, BE, CH, DE, DK, FR, GB, IT, LI, LU, NL, PT, SE)

1. A solution with factor VIII:C activity greater than 1000 IU/mg, which is stabilized by addition of arginine or its salts and, where appropriate, contains as further constituents a detergent and/or an organic polymer.

2. A solution as claimed in claim 1, which additionally contains glycine.

3. A solution as claimed in claim 1 or 2, wherein arginine and glycine are present each in the range from 0.01 to 1 mol/l together with Tween® 80 in the range from 0.001% to 0.5% (v/v) and sucrose in the range from 0.1% to 10%.

4. A solution as claimed in claim 3, wherein 0.14 M arginine, 0.4 M glycine, 0.05% Tween® 80, 1% sucrose and 0.1 M sodium chloride are present.

5. A method for stabilizing a factor VIII:C solution with factor VIII:C activity greater than 1000 IU/mg, which comprises adding arginine or its salts and, where appropriate, as further constituents a detergent and/or an organic polymer.

6. A pharmaceutical containing a solution as claimed in any of claims 1, 2, 3 or 4.

## Claims (Claims for the following Contracting State(s): ES, GR)

1. A method for stabilizing a factor VIII:C solution with factor VIII:C activity greater than 1000 IU/mg, which comprises adding arginine or its salts and, where appropriate, as further constituents a detergent and/or an organic polymer.

2. The method as claimed in claim 1, wherein glycine is added in addition.

3. The method as claimed in claim 1 or 2, wherein arginine and glycine are added each in the range from 0.01 to 1 mol/l together with Tween® 80 in the range from 0.001% to 0.5% (v/v) and sucrose in the range from 0.1% to 10%.

4. The method as claimed in claim 3, wherein 0.14 M arginine, 0.4 M glycine, 0.05% Tween® 80 and 1% sucrose and 0.1 M sodium chloride are added.

## Revendications (Revendications pour l'(les) Etat(s) contractant(s) suivant(s): AT, BE, CH, DE, DK, FR, GB, IT, LI, LU, NL, PT, SE)

1. Solution avec une activité du facteur VIII C supérieure à 1000 UI/mg, qui est stabilisée par ajout d'arginine ou de ses sels et contient éventuellement comme autre constituant un tensioactif et/ou un polymère organique.

2. Solution selon la revendication 1, caractérisée en ce qu'elle contient en plus de la glycine.

3. Solution selon la revendication 1 ou 2, caractérisée en ce qu'elle contient de l'arginine et de la glycine à raison respectivement de 0,01 jusqu'à 1 mol/l, avec en même temps du Tween® 80 à raison de 0,001% jusqu'à 0,5% (v/v) et du saccharose à raison de 0,1% jusqu'à 10%.

4. Solution selon la revendication 3, caractérisée en ce qu'elle contient 0,14 M d'arginine, 0,4 M de glycine, 0,05% de Tween® 80, 1% de saccharose et 0,1 M de chlorure de sodium.

5. Procédé de stabilisation d'une solution du facteur VIII:C avec une activité du facteur VIII:C supérieure à 1000 UI/mg, caractérisé en ce que l'on ajoute de l'arginine ou de ses sels et éventuellement comme autre constituant un tensioactif et/ou un polymère organique.

6. Médicament contenant une solution selon les revendications 1, 2, 3 ou 4.

## Revendications (Revendications pour l'(les) Etat(s) contractant(s) suivant(s): ES, GR)

1. Procédé de stabilisation d'une solution du facteur VIII:C avec une activité du facteur VIII:C supérieure à 1000 UI/mg, caractérisé en ce que l'on ajoute de l'arginine ou de ses sels et éventuellement comme autre constituant un tensioactif et/ou un polymère organique.

2. Procédé selon la revendication 1, caractérisé en ce que l'on ajoute en plus de la glycine.

3. Procédé selon la revendication 1 ou 2, caractérisé en ce qu'on ajoute de l'arginine et de la glycine à raison respectivement de 0,01 jusqu'à 1 mol/l, avec en même temps du Tween® 80 à raison de 0,001% jusqu'à 0,5% (v/v) et du saccharose à raison de 0,1% jusqu'à 10%.

4. Procédé selon la revendication 3, caractérisé en ce qu'on ajoute 0,14 M d'arginine, 0,4 M de glycine, 0,05% de Tween® 80, 1% de saccharose et 0,1 M de chlorure de sodium.
